# EUROPEAN PATENT APPLICATION

(11) **EP 2 269 646 A1**
(43) Date of publication of application: **05.01.2011**
(21) Application number: 09715914.9
(22) Date of filing: 25.02.2009
(51) Int. Cl.: A61K 45/00, A61K 38/00, A61P 3/04, A61P 3/10, A61P 43/00

(54) **PROPHYLACTIC OR THERAPEUTIC AGENT FOR DIABETES OR OBESITY**

(30) Priority: 25.02.2008 JP 2008043348
(71) Applicant: Ajinomoto Co., Inc., Tokyo 104-8315 (JP); National University Corporation Hokkaido University, Sapporo-shi, Hokkaido 060-0808 (JP)
(72) Inventor: HARA, Hiroshi, Hokkaido 060-8589 (JP); HIRA, Tohru, Hokkaido 060-8589 (JP); ETO, Yuzuru, Kawasaki-shi Kanagawa 210-8681 (JP)
(74) Representative: Fischer, Heinrich
(86) International application number: PCT/JP2009/053409
(87) International publication number: WO 2009/107660

(57) **Abstract**

Disclosed is a safe prophylactic or therapeutic agent for diabetes or obesity. The prophylactic or therapeutic agent for diabetes or obesity comprises, as an active ingredient, a calcium receptor activator such as γ-Glu-X-Gly [wherein X represents an amino acid or an amino acid derivative], γ-Glu-Val-Y [wherein Y represents an amino acid or an amino acid derivative], γ-Glu-Ala, γ-Glu-Gly, γ-Glu-Cys, γ-Glu-Met, γ-Glu-Thr, γ-Glu-Val, γ-Glu-Om, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), cinacalcet, a cinacalcet analogue compound and protamine.

## Description

### Technical Field

The present invention relates to a prophylactic or therapeutic agent for diabetes or obesity, comprising a peptide or the like having a calcium receptor-activating function as an active ingredient.

### Background Art

In modem society, obesity, hypertension, impaired glucose tolerance, and dyslipidemia are said to be four major diseases owing to the increased prevalence of a high-fat diet and a high-calorie diet. As a result, the incidence of heart disease or arteriosclerosis increases, which puts our lives at risk. Therefore, there is a demand for development of an effective method for prophylaxis or treatment of diabetes or obesity.

*In vivo* energy metabolism is controlled by insulin which is produced in pancreatic β cells. Insulin acts on peripheral tissues and cells and promotes the intake of sugar from blood, to thereby play an important role in the control of blood-sugar level. However, when the insulin sensitivity of cells is lowered by the continuous intake of a high-calorie diet, the elevation of blood-sugar level is proportionate to the excess secretion of insulin. As a result, the pancreatic β cells become exhausted and dysfunctional, leading to the development of diabetes and obesity.

The secretion of insulin is regulated by various hormones, and in particular, glucagon-like peptide-1 (GLP-1) which is produced and secreted in the gastrointestinal tract, and is considered to be important GLP-1 is a peptide hormone having a molecular weight of about 4000, and is mainly produced in L cells of the small intestine. It has been reported that GLP-1 has, for example, functions of promoting the secretion of insulin from β cells, suppressing gastric emptying, and suppressing appetite or overeating, and is effective for treatment and prophylaxis of diabetes and obesity. It is known that a decline in ability of producing GLP-1 is observed in diabetes and obesity. Thus, the promotion of production of GLP-1 in those conditions is expected to lead to treatment and prophylaxis of diabetes and obesity. The production of GLP-1 in L cells is promoted by the intake of various nutrients such as carbohydrates, lipids, and proteins, but an example where a compound such as a peptide of a specific ingredient is employed as a GLP-1 secretion promoter is rare.

Cholecystokinin (CCK) is a peptide hormone having a molecular weight of about 4000, is mainly produced in L cells of the duodenum and the small intestine, and promotes the secretion of bile and the secretion of pancreatic digestive juice. Particularly noticeable physiological function of CCK are functions of suppressing the gastric emptying of foods, promoting the secretion of pancreatic enzymes, and suppressing food intake through a sensation of fullness (Non-patent Documents 1 and 2). In addition, it is also known CCK has a function of promoting the secretion of insulin as a blood sugar-regulating hormone (Non-patent Documents 3 and 4). CCK has such functions, and hence is considered to be promising for treatment or prophylaxis of lifestyle-related diseases such as diabetes, obesity, and pancreatitis.

GLP-1 and CCK are peptide hormones. Thus, in a therapeutic application, GLP-1 and CCK are administered into blood by injection or the like, which is not realistic in terms of the complication of daily administration and the considerable expense. Meanwhile, it is conceivable to utilize such a mechanism that endogenous GLP-1 and CCK are secreted from GLP-1- and CCK-producing cells present in the small intestinal mucosa by using proteins, peptides, amino acids, fatty acids, and the like as food ingredients. That is, there has been a demand for development of a compound or a food material having a function of promoting the secretion of GLP-1 and CCK.

The calcium receptor is also called Calcium Sensing Receptor (CaSR), and is formed of 1078 amino acids, which is classified into the class C seven-transmembrane receptors (G protein-coupled receptor). Cloning of the gene for the calcium receptor was reported in 1993 (Non-patent document 5), and the calcium receptor is known to cause various cell responses through elevation of intracellular calcium level or the like, when being activated with calcium or the like. The gene sequence of the human calcium receptor is registered with GenBank Accession No. NM_000388 and is well conserved in animals.

In addition to calcium, cations such as a gadolinium cation, basic peptides such as polyarginine, polyamines such as spermine, proteins such as protamine, amino acids such as phenylalanine, and so forth have been reported as calcium receptor activators (Non-patent document 6).

Non-patent Document 7 reports that glutathione (γ-Glu-Cys-Gly) as a low molecular weight peptide is a calcium receptor activator (that is, has an activating function), but does not disclose that glutathione can be effective for prophylaxis or treatment of diabetes and obesity.

Meanwhile, Patent Document 1 discloses that a dipeptide or a tripeptide having a specific sequence is effective as a calcium receptor activator, describes a possibility for the dipeptide or the tripeptide to be used as a therapeutic drug for various diseases, but does not disclose that the dipeptide or the tripeptide is effective for diabetes and obesity.
Patent Document 1: WO 2007/055388 A1
Non-patent Document 1: Science, vol.247, p.1589-1591, 190
Non-patent Document 2: American Journal of Physiology, vol.276, R1701-R1709, 1999
Non-patent Document 3: Diabetes, vol.36, p.1212-1215, 1987
Non-patent Document 4: Journal of Clinical Endocrinological Metabolism, vol.65, p.395-401, 1987
Non-patent Document 5: Nature, 1993, Vol.366 (6455), p.575-580
Non-patent Document 6: Cell Calcium, 2004, Vol.35 (3), p.209-216
Non-patent Document 7: J. Biol. Chem., 2006, Vol.281 (13), p.8864-8870

### Disclosure of the Invention

As described above, lifestyle-related diseases, in particular, diabetes and obesity have become a serious social issue. For prophylaxis or treatment of those lifestyle-related diseases, there is a need for development of pharmaceuticals, or foods or useful substances equivalent to the foods, which are highly safe for the living body. An object of the present invention is to provide a prophylactic or therapeutic agent for diabetes, obesity, or both, which promotes the production of GLP-1, CCK, or both in a gastrointestinal tract tissue, and which is highly safe for the living body.

The inventors of the present invention have studied a calcium receptor activator, and have found that the calcium receptor activator has a function of promoting the secretion of GLP-1 and CCK from GLUTag and STC-1 cells derived from the intestinal tract. To be more specific, the inventors have found that a peptide or a low molecular weight compound having a calcium receptor-activating function and being used in examples mentioned below promotes the secretion of GLP-1 and CCK, and thus can serve as a prophylactic or therapeutic agent for diabetes and obesity, and have completed the present invention.

That is, the present invention relates to the followings:
(1) a prophylactic or therapeutic agent for diabetes or obesity, comprising a calcium receptor activator,
(2) the prophylactic or therapeutic agent for diabetes or obesity, wherein the calcium receptor activator is one kind or two or more kinds selected from the group consisting of γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Om, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-CyS(S-Me)(O), γ-Glu-Leu, γ-Glu-Ile, γ-Glu-t-Leu, γ-Glu-Cys(S-Me), cinacalcet, and an analogous compound of cinacalcet;
(3) the prophylactic or therapeutic agent for diabetes or obesity, wherein X represents Cys(SNO), Cys(S-allyl), Gly, Cys(S-Me), Abu, or Ser, and Y represents Gly, Val, Glu, Lys, Phe, Ser, Pro, Arg, Asp, Met, Thr, His, Orn, Asn, Cys, or Gln;
(4) the prophylactic or therapeutic agent for diabetes or obesity, wherein the calcium receptor activator is γ-Glu-Val-Gly, γ-Glu-Cys-Gly, or cinacalcet;
(5) the prophylactic or therapeutic agent for diabetes or obesity, wherein the calcium receptor activator is γ-Glu-Cys;
(6) the prophylactic or therapeutic agent for diabetes or obesity, wherein the calcium receptor activator is protamine;
(7) a food for prophylaxis or treatment of diabetes or obesity, comprising γ-Glu-Cys-Gly in an amount of 0.00000 1 % or more;
(8) a food for prophylaxis or treatment of diabetes or obesity, comprising γ-Glu-Cys in an amount of 0.000001% or more;
(9) a food for prophylaxis or treatment of diabetes or obesity, comprising protamine in an amount of 0.000001% or more;

(10) a use of a calcium receptor activator for production of a prophylactic or therapeutic agent for diabetes or obesity;
(11) the use, wherein the calcium receptor activator is one kind or two or more kinds selected from the group consisting of γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Om, Asp-Crly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), cinacalcet, and an analogous compound of cinacalcet;
(12) the use, wherein X represents Cys(SNO), Cys(S-allyl), Gly, Cys(S-Me), Abu, or Ser, and Y represents Gly, Val, Glu, Lys, Phe, Ser, Pro, Arg, Asp, Met, Thr, His, Om, Asn, Cys, or Gln;
(13) the use, wherein the calcium receptor activator is γ-Glu-Val-Gly, γ-Glu-Cys-Gly, or cinacalcet;
(14) the use, wherein the calcium receptor activator is γ-Glu-Cys;
(15) the use, wherein the calcium receptor activator is protamine;

(16) a method for prophylaxis or treatment of diabetes or obesity, comprising administering a calcium receptor activator to a subject in need of prophylaxis or treatment of diabetes or obesity;
(17) the method, wherein the calcium receptor activator is one kind or two or more kinds selected from the group consisting of γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Om, Asp-Gly, Cys-Gly, Cys-Met, Glu-Cys, Gly-Cys, Leu-Asp, γ-Glu-Met(O), γ-Glu-γ-Glu-Val, γ-Glu-Va-NH₂, γ-Glu-Val-ol, γ-Glu-Ser, γ-Glu-Tau, γ-Glu-Cys(S-Me)(O), γ-Glu-Leu, γ-Glu-lle, γ-Glu-t-Leu, γ-Glu-Cys(S-Me), cinacalcet, and an analogous compound of cinacalcet.
(18) the method, wherein X represents Cys(SNO), Cys(S-allyl), Gly, Cys(S-Me), Abu, or Ser, and Y represents Gly, Val, Glu, Lys, Phe, Ser, Pro, Arg, Asp, Met, Thor, His, Om, Asn, Cys, or Gln;
(19) the method, wherein the calcium receptor activator is γ-Glu-Val-Gly, γ-Glu-Cys-Gly, or cinacalcet;
(20) the method, wherein the calcium receptor activator is γ-Glu-Cys; and
(21) the method, wherein the calcium receptor activator is protamine.

### Brief Description of the Drawings

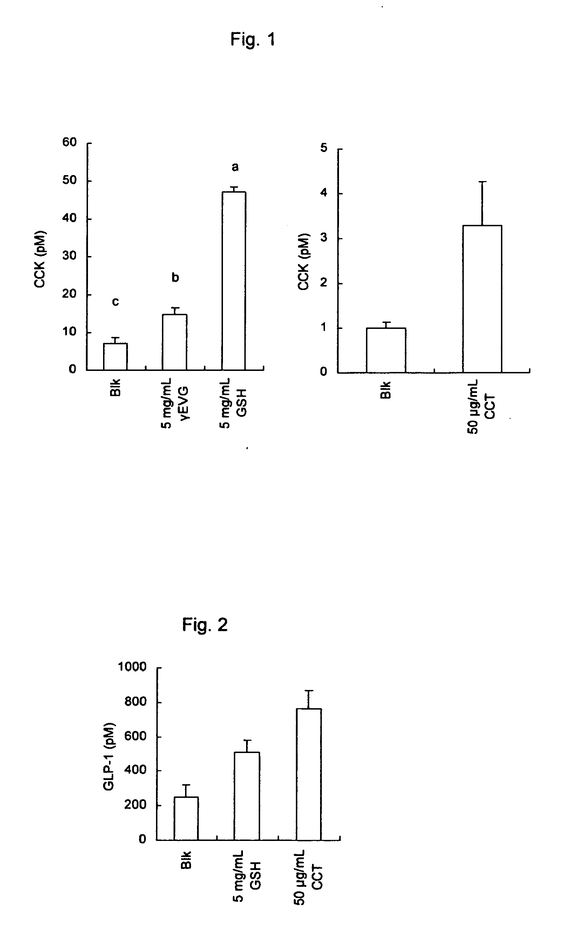

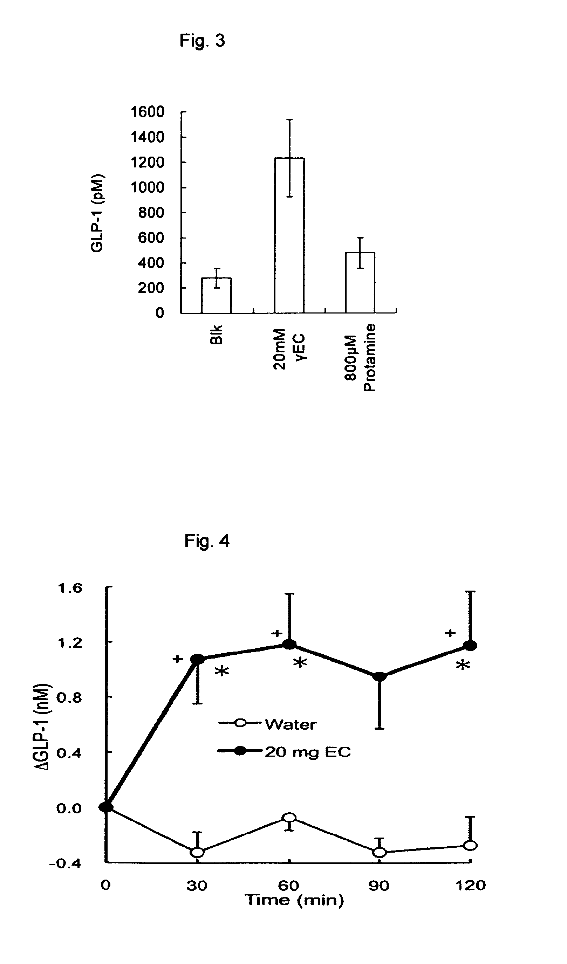

FIGS. 1 are graphs each illustrating the promotion of secretion of CCK from STC-1 cells by γ-Glu-Cys-Gly, γ-Glu-Val-Gly, and cinacalcet. In the graphs, Blk, yEVG, GSH, and CCT represent a blank (control), γ-Glu-Val-Gly, γ-Glu-Cys-Gly, and cinacalcet, respectively. The concentrations represent final concentrations in wells. The same is applied in FIGS. 2 and 3.
FIG. 2 is a graph illustrating the promotion of secretion of GLP-1 from GLUTag cells by γ-Glu-Cys-Gly and cinacalcet.
FIG. 3 is a graph illustrating the promotion of secretion of GLP-1 from GLUTag cells by γ-Glu-Cys and protamine. In the graph, γEC represents γ-Glu-Cys.
FIG. 4 is a graph illustrating a change in GLP-1 secretion from the rat intestinal tract by γ-Glu-Cys (EC). The values are plotted as average values with standard errors (n=8). The symbol "+" means that there is a statistically significant difference with respect to 0 minute, and the symbol "*" means that there is a statistically significant difference with respect to a water administration group (Tukey's test, P<0.05).

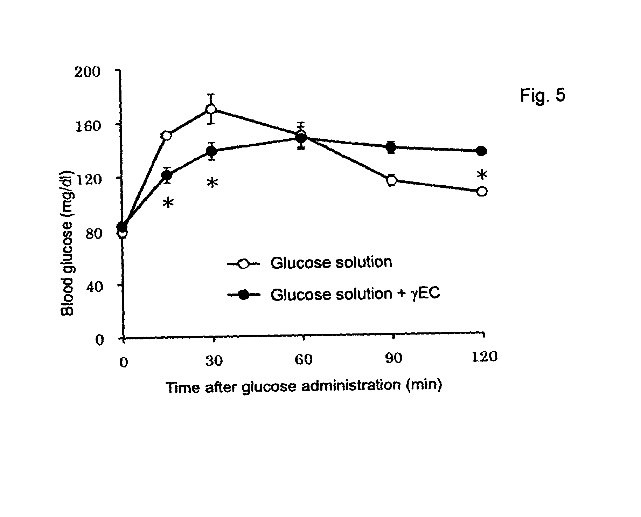

FIG. 5 is a graph illustrating the effect of suppressing the elevation of blood sugar by γ-Glu-Cys (EC) in a rat. The values are plotted as average values with standard errors (n=8). The symbol "*" means that there is a statistically significant difference with respect to a glucose solution administration group (Tukey's test, P<0.05)

### Description of the Preferred Embodiments

### Hereinafter, the present invention is described in detail.

A prophylactic or therapeutic agent for diabetes or obesity of the present invention comprises a calcium receptor activator as an active ingredient. The calcium receptor activator is preferably a peptide or a low molecular weight compound having a calcium receptor-activating function.

The term "calcium receptor" as used herein refers to a receptor that is called Calcium Sensing Receptor (CaSR) and belongs to the class C seven-transmembrane receptors. The term "calcium receptor activator" as used herein refers to a substance that binds to the above-mentioned calcium receptor to activate the calcium receptor and control functions of cells which express the calcium receptor. Further, the phrase "to activate a calcium receptor" as used herein means that a ligand binds to a calcium receptor to activate a guanine nucleotide binding protein, to thereby transmit a signal. In addition, the term "calcium receptor activity" means that the calcium receptor transmits the signal.

### <1> Peptide or low molecular weight compound having calcium receptor-activating function

The peptide or the low molecular weight compound having a calcium receptor-activating function may be obtained, for example, by reacting a calcium receptor and a test substance with each other, and detecting a calcium receptor activity It is preferably to confirm whether the thus obtained peptide or low molecular weight compound has a function of promoting the secretion of GLP-1 or CCK, or a prophylactic or therapeutic effect on diabetes or obesity.

Hereinafter, a method of screening the peptide or the low molecular weight compound having a calcium receptor-activating function is specifically described, but is not limited to the steps:
1) measuring a calcium receptor activity by adding a test substance to a calcium receptor activity measurement system for measuring the calcium receptor activity;
2) comparing a calcium receptor activity with the test substance to a calcium receptor activity without the test substance; and
3) selecting the test substance exhibiting a high calcium receptor activity with the test substance.

The calcium receptor activity is, for example, measured by using a measurement system using cells that express calcium receptors. The above-mentioned cells may be cells endogenously expressing calcium receptors, or may be recombinant cells wherein exogenous calcium receptor genes are introduced. The above-mentioned calcium receptor activity measurement system may be used without any particular limitation as long as, when an extracellular ligand (activator) specific to a calcium receptor is added to the above-mentioned cells that express calcium receptors, the measurement system can detect the binding (reaction) between the activator and the calcium receptor, or can respond to the binding (reaction) between the activator and the calcium receptor to thereby transmit a detectable signal into the cells. When the calcium receptor activity is detected through the reaction with the test substance, it is confirmed that the test substance has a calcium receptor-stimulating activity, and is a substance having a prophylactic or therapeutic agent for diabetes or obesity.

Meanwhile, the prophylactic or therapeutic effect on diabetes or obesity, which the calcium receptor activator possesses, may be examined by, for example, such a test for examining a function of promoting the secretion of GLP-1 or CCK as described in examples. Further, the prophylactic or therapeutic effect on diabetes may also be confirmed by, for example, such a glucose tolerance test for examining an effect of suppressing the elevation of blood sugar as described in examples. Further, the peptide and the low molecular weight compound to be used as a test substance is not particularly limited, and the peptide is preferably a peptide formed of 2 to 10 amino acid residues or a derivative thereof, or more preferably a peptide formed of 2 or 3 amino acid residues or a derivative thereof Further, the amino acid residue at the N-terminal side of the peptide is preferably γ-glutamic acid. The low molecular weight compound is preferably cinacalcet
((R)-N-(3-(3-(trmuoromethyl)phenyl)propyl)-1-(1-naphthyl)ethylamine) or an analogous compound thereof. The analogous compound of cinacalcet is mentioned later.

The origin of the above-mentioned calcium receptor is not particularly limited. Examples thereof include not only the above-mentioned human calcium receptor, but also a calcium receptor derived from an animal such as a mouse, a rat, and a dog. Specifically, preferred examples of the above-mentioned calcium receptor may include the human calcium receptor encoded by the human calcium receptor gene registered with GenBank Accession No NM_00388. It should be noted that the calcium receptor is not limited to the protein encoded by the gene having the above-mentioned sequence, and may be a protein encoded by a gene having a 60% or more, preferably 80% or more, and more preferably 90% or more homology to the above-mentioned sequence as long as the gene encodes a protein having a calcium receptor function. The GPRC6A receptor or 5.24 receptor is also known as a subtype of the calcium receptor, and may be used in the present invention. It should be noted that the calcium receptor function may be investigated by expressing those genes in cells, and measuring a change in current at the time of the addition of calcium, and the change in the intracellular calcium ion concentration.

As described above, the calcium receptor activity may be confirmed by using live cells expressing a calcium receptor or its fragment, cell membranes expressing a calcium receptor or its fragment, an *in vitro* system containing a protein of a calcium receptor or its fragment, or the like.
An example using live cells is described below. However, confirmation of the calcium receptor activity is not limited to this example.

A calcium receptor is expressed in cultured cells such as *Xenopus laevis* oocytes, hamster ovarian cells, and human fetal kidney cells. The calcium receptor can be expressed by cloning a calcium receptor gene in a plasmid that carries a foreign gene and introducing the plasmid or cRNA obtained by using the plasmid as a template. To detect the reaction, an electrophysiological technique and a fluorescent indicator that indicates an increase in intracellular calcium level may be used.

Expression of the calcium receptor is first confirmed based on the response to calcium or a specific activator. Oocytes showing intracellular current with calcium at a concentration of about 5 mM, or cultured cells showing fluorescence of the fluorescent indicator reagent with calcium at a concentration of about 5 mM are used. The calcium concentration dependency is determined by changing the calcium concentration. Then, a test substance such as a peptide is added to the oocytes or cultured cells to a concentration of about 1 µM to 1 mM, and the calcium receptor activity of the above-mentioned peptide or the like is determined.

Examples of the peptide having a calcium receptor-activating activity to be used in the present invention include γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Crlu-Val, γ-Glu-Om, Asp-Crly, Cys-Gly, Cys-Met, Glu-Cys, Gly-Cys, Leu-Asp, γ-Glu-Met(O), γ-Glu-y-Glu-Val, γ-Glu-Val-NH2, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me) (hereinafter, also referred to as a "peptide of the present invention".

Of the above-mentioned compounds, a preferred compound are those wherein X represents Cys, Cys(SNO), Cys(S-allyl), Gly, Cys(S-Me), Abu, or Ser, and Y represents Gly, Val, Glu, Lys, Phe, Ser, Pro, Arg, Asp, Met, Thr, His, Om, Asn, Cys, or Gln.

It should be noted that, in this description, an amino acid of which each peptide is formed is an L-amino acid unless otherwise stated. Herein, examples of the amino acid include: a neutral amino acid such as Gly, Ala, Val, Leu, Ile, Ser, Thr, Cys, Met, Asn, Gln, Pro, and Hyp; an acidic amino acid such as Asp and Glu; a basic amino acid such as Lys, Arg, and His; an aromatic amino acid such as Phe, Tyr, and Trp; and homoserine, citrulline, omithine, α-aminobutyric acid, norvaline, norleucine, and taurine.

In this description, abbreviations for amino group residues mean the following amino acids.
(1) Gly: Glycine
(2) Ala: Alanine
(3) Val: Valine
(4) Leu: Leucine
(5) Ile: Isoleucine
(6) Met: Methionine
(7) Phe: Phenylalanine
(8) Tyr: Tyrosine
(9) Trp: Tryptophan
(10) His: Histidine
(11) Lys: Lysine
(12) Arg: Arginine
(13) Ser: Serine
(14) The Threonine
(15) Asp: Aspartic acid
(16) Glu: Glutamic acid
(17) Asn: Asparagine
(18) Gln: Glutamine
(19) Cys: Cysteine
(20) Pro: Proline
(21) Orn: Ornithine
(22) Sar: Sarcosine
(23) Cit: Citrulline
(24) N-Val: Norvaline
(25) N-Leu: Norleucine
(26) Abu: α-Aminobutyric acid
(27) Tau: Taurine
(28) Hyp: Hydroxyproline
(29) t-Leu: tert-Leucine

Further, examples of the amino acid derivative include various derivatives of the above-mentioned amino acids such as an unusual amino acid, a non-natural amino acid, an amino alcohol, and a substituted amino acid where an amino acid side chain such as the terminal carbonyl group, the terminal amino group, and the thiol group of cysteine, is substituted with various substituents. Examples of the substituent include an alkyl group, an acyl group, a hydroxy group, an amino group, an alkylamino group, a nitro group, a sulfonyl group, and various protection groups. Examples of the substituted amino acid include: Arg(NO₂): N-γ-nitroarginine; Cys(SNO): S-nitrocysteine; Cys(S-Me): S-methylcysteine; Cys(S-allyl): S-allylcysteine; Val-NH₂: valinamide; and Val-ol: valinol (2-amino-3-methyl-1-butanol).

It should be noted that γ-Glu-Cys(SNO)-Gly described above has the following structural formula, and the "(O)" in the above-mentioned formulae γ-Glu-Met(O) and γ-Glu-Cys(S-Me)(O) indicates a sulfoxide structure. The "γ" in γ-Glu indicates that the glutamic acid binds to another amino acid via the carboxy group at the γ position of the glutamic acid.

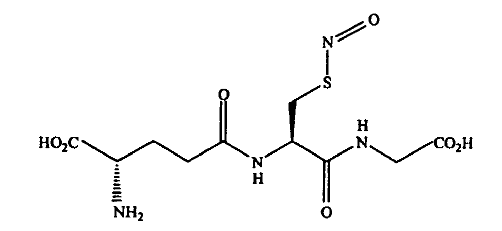

S-Nitrosoglutathione (GNSO)

It has been shown that γ-Crlu-X-Gly where X represents an amino acid or an amino acid derivative, γ-Glu-Val-Y where Y represents an amino acid or an amino acid derivative, γ-Glu- Ala, γ-Glu-Gly, γ-Glu-Cys, γ-Glu-Met, γ-Glu-Thr, γ-Glu-Val, γ-Glu-Om, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-MeXO), γ-Glu-Leu, γ-Glu-lle, γ-Glu-t-Leu, and γ-Glu-Cys(S-Me) activate a calcium receptor (WO 2007/055388 A1, WO 2007/055393 A1, WO 2008/139945 A1, WO 2008/139946 A1, and WO 2008/139947 A1). Further, as illustrated in the Examples, it has been found that a plurality of kinds of compounds having a calcium receptor-activating function promotes the secretion of GLP-1 or CCK. Thus, γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Om, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me) may be used as a prophylactic or therapeutic agent for diabetes or obesity. In the present invention, the peptide or the low molecular weight compound to be used for the present invention may be used alone, or may be used as a mixture of any two kinds or three or more kinds thereof

Of the above-mentioned compounds, particularly preferred compounds are γ-Glu-Val-Gly, γ-Glu-Cys-Gly, and cinacalcet. Further, γ-Glu-Cys is also particularly preferred. γ-Glu-Val-Gly, γ-Glu-Cys-Gly, and cinacalcet are particularly suitably used as prophylactic or therapeutic agents for obesity, and γ-Glu-Cys, γ-Glu-Cys-Gly, and cinacalcet are particularly suitably used as prophylactic or therapeutic agents for diabetes. The present invention includes, for example, following:
prophylactic or therapeutic agents for obesity comprising γ-Glu-Val-Gly as an active ingredient;
prophylactic or therapeutic agents for obesity comprising γ-Glu-Cys-Gly as an active ingredient;
prophylactic or therapeutic agents for obesity comprising cinacalcet as an active ingredient;
prophylactic or therapeutic agents for diabetes comprising γ-Glu-Cys as an active ingredient;
prophylactic or therapeutic agents for diabetes comprising γ-Glu-Cys-Gly as an active ingredient; and
prophylactic or therapeutic agents for diabetes comprising cinacalcet as an active ingredient

A commercially available product may be used as the above-mentioned peptide. Further, the peptide may be obtained by appropriately employing a known technique such as (1) a chemical synthesis method or (2) an enzymatic synthesis method. The peptide to be used in the present invention contains 2 to 3 amino acid residues, i.e., is relatively short, and hence, the chemical synthesis method is convenient. In the case of the chemical synthesis method, the oligopeptide may be synthesized or semi-synthesized by using a peptide synthesizer. An example of the chemical synthesis method includes a peptide solid phase synthesis method. The peptide synthesized as described above may be purified by general means such as ion exchange chromatography, reversed phase high performance liquid chromatography, or affinity chromatography. Such peptide solid phase synthesis method and the subsequent peptide purification are well known in the technical field.

Further, the peptide to be used in the present invention may also be produced by an enzymatic reaction. For example, the method described in WO 2004/011653 A1 can be used. That is, the peptide may also be produced by reacting one amino acid or dipeptide whose carboxyl terminus is esterified or amidated and an amino acid having a free amino group (for example, an amino acid whose carboxyl group is protected) in the presence of a peptide-producing enzyme, and purifying the produced dipeptide or tripeptide. Examples of the peptide-producing enzyme include a culture of a microorganism having an ability of producing a peptide, microbial cells separated from the culture, or a processed product of the microbial cells, or a peptide-producing enzyme derived from the microorganism.

It should be noted that the peptide to be used in the present invention is not only produced by such an enzymatic method or a chemical synthesis method as mentioned above, but also may exist in, for example, a plant such as a vegetable or a fruit, a microorganism such as a yeast, and a yeast extract. When the peptide exists in natural products, the peptide may be extracted from those natural products before use.

Further, the peptide does not need to be isolated before use, and there may be used a fraction containing the peptide of the present invention in a large amount For example, there is exemplified a yeast extract containing glutathione (γ-Glu-Cys-Gly) or a fraction thereof. The preparation of the yeast extract or the like may be conducted in the same manner as general yeast extract preparation. The yeast extract may be a treated product of yeast cells extracted with hot water, or may be a treated product of digested yeast cells. The fraction of the yeast extract is not particularly limited as long as the fraction contains glutathione. Further, for example, there is also exemplified a yeast extract containing γ-Glu-Cys or a fraction thereof. The preparation of the yeast extract or the like may be conducted in the same manner as general yeast extract preparation. The yeast extract may be a treated product of yeast cells extracted with hot water, or may be a treated product of digested yeast cells. The fraction of the yeast extract is not particularly limited as long as the fraction contains γ-Glu-Cys.

The peptide to be used in the present invention also include that in the form of a salt When the peptide of the present invention is in the form of a salt, the salt may be a pharmacologically acceptable salt Examples of a salt with an acidic group such as a carboxyl group in the formula include: an ammonium salt; a salt with an alkali metal such as sodium and potassium; a salt with an alkaline earth metal such as calcium and magnesium; an aluminum salt; a zinc salt; a salt with an organic amine such as triethylamine, ethanolamine, morpholine, pyrrolidine, piperidine, piperazine, and dicyclohexylamine; and a salt with a basic amino acid such as arginine and lysine. Examples of a salt with a basic group in a case where the basic group exists in the formula include: a salt with an inorganic acid such as hydrochloric acid, sulfuric acid, phosphoric acid, nitric acid, and hydrobromic acid; a salt with an organic carboxylic acid such as acetic acid, citric acid, benzoic acid, maleic acid, fumaric acid, tartaric acid, succinic acid, tannic acid, butyric acid, hibenzoic acid, pamoic acid, enanthoic acid, decanoic acid, teoclic acid, salicylic acid, lactic acid, oxalic acid, mandelic acid, and malic acid; and a salt with an organic sulfonic acid such as methanesulfonic acid, benzenesulfonic acid, and p-toluenesulfonic acid.

Examples of the low molecular weight compounds having a calcium receptor activity to be used in the present invention include cinacalcet and analogous compounds of cinacalcet. Examples of the analogous compounds of cinacalcet include compounds described in US 6211244 A, US 6213146 A, US 5688938 A, US 5763569 A, US 5858684 A, US 5962314 A, US 6001884 A, US 6011068 A, US 6031003 A, WO 1995/011221 A1, WO 1996/012687 A1, WO 2002/059102 A1, and the like. It should be noted that, as in the case of the above-mentioned peptide, the low molecular weight compound to be used in the present invention also includes that in the form of a salt.

### <2> Prophylactic or therapeutic agent for diabetes or obesity

The peptide and the low molecular weight compound each having a calcium receptor-activating function may be used as active ingredients in the prophylactic or therapeutic agent for diabetes or obesity In the present invention, the calcium receptor activators may be used alone, or may be used as a mixture of any two kinds or three or more kinds thereof. Examples of the form of the prophylactic or therapeutic agent for diabetes or obesity of the present invention include pharmaceuticals, quasi drugs, and foods.

A method of applying the prophylactic or therapeutic agent for diabetes or obesity of the present invention is not particularly limited, and an oral administration, an invasive administration utilizing an injection or the like, a suppository administration, or a transdermal administration may be adopted. The prophylactic or therapeutic agent for diabetes or obesity of the present invention may be administered in the form of a conventionally-used pharmaceutical formulation by mixing its active ingredient with a solid or liquid non-toxic pharmaceutical carrier, which is suitable for an administration method such as an oral administration and an injection. In the present invention, an oral administration is particularly preferably adopted. Examples of such a formulation include: a form of a solid formulation such as a tablet, a granule, a powder, and a capsule; a form of a liquid formulation such as a solution, a suspension, and an emulsion; and a form of a lyophilizate or the like. Those formulations may be prepared by usual methods for preparing the formulations.

Examples of the above-mentioned non-toxic pharmaceutical carrier include glucose, lactose, sucrose, starch, mannitol, dextrin, fatty acid glyceride, polyethylene glycol, hydroxyethyl starch, ethylene glycol, polyoxyethylene sorbitan fatty acid ester, gelatin, albumin, amino acid, water, and physiological saline. Further, if required, a conventionally-used additive agent such as a stabilizing agent, a wetting agent, an emulsifier, a binder, and a tonicity agent may be appropriately added.

Further, the prophylactic or therapeutic agent for diabetes or obesity of the present invention may comprise, in addition to the peptide and/or the low molecular weight compound, one kind or two or more kinds of other calcium receptor activators.

Examples of the above-mentioned other calcium receptor activators include, but not limited to, a cation such as a calcium cation and a gadolinium cation; a basic peptide such as polyarginine and polylysine; a polyamine such as putrescine, spermine, and spermidine; a protein such as protamine; an amino acid such as phenylalanine. In the present invention, the calcium receptor activator may be used alone, or may be used as a mixture of any two kinds or three or more kinds thereof. Of the above-mentioned other calcium receptor activators, preferred is a cation such as a calcium cation and a gadolinium cation, and more preferred is a calcium cation. In other words, at least one kind of the other calcium receptor activators to be further added is preferably a cation.

When the above-mentioned other calcium receptor activators coexist with the peptide or the low molecular weight compound, stronger activation on a calcium receptor is observed. The ratio of the total of the peptide and the low molecular weight compound of the present invention to the total of other calcium receptor activators in the prophylactic or therapeutic agent for diabetes or obesity of the present invention is not particularly limited as long as a stronger activation on the calcium receptor is attained. For example, the mass ratio of the total of the other calcium receptor activators to the total of the peptide or the low molecular weight compound is preferably set to 1:100 to 100:1.

The dosage or intake of the prophylactic or therapeutic agent for diabetes or obesity of the present invention may be any amount as long as the amount is effective for therapy or prophylaxis, and is appropriately adjusted depending on the age, gender, body weight, symptom, and the like of a patient. For example, in the case of an oral administration, the total amount of the peptide and the low molecular weight compound to be used in the present invention is preferably 0.01 g to 10 g per kg body weight per one dose and more preferably 0.1 g to 1 g per kg body weight per one dose. The administration frequency is not particularly limited, and the administration may be performed once to several times per day.

The content of the peptide or the low molecular weight compound in the prophylactic or therapeutic agent for diabetes or obesity of the present invention is not particularly limited as long as the content is suited to the above-mentioned dosage. The content is preferably 0.000001% by mass to 99.9999% by mass, more preferably 0.00001 % by mass to 99.999% by mass, and particularly preferably 0.0001% by mass to 99.99% by mass with respect to the dry weight

The prophylactic or therapeutic agent for diabetes or obesity of the present invention may also be used as a food having a therapeutic or prophylactic effect on diabetes or obesity, for example, a food in a container or a package indicating that the agent has a prophylactic or therapeutic effect on diabetes or obesity. The food comprises the peptide or the low molecular weight compound in an amount of preferably 0.000001% or more, more preferably 0.00001 % or more, or particularly preferably 0.00001 % or more and 98% or less. The form of the food is not particularly limited, and the food may be produced in the same production method as a general food and with the same materials as those for the general food except that the peptide or the low molecular weight compound is blended. Examples of the food include a seasoning, a beverage, a health food, a processed agricultural product, a processed fishery product, and a processed animal product.

### <3> Protamine

As the calcium receptor activator comprised as an active ingredient in the prophylactic or therapeutic agent for diabetes or obesity of the present invention, protamine is also preferred. The above detailed description on the case of the peptide or the low molecular weight compound is also basically applied mutatis mutandis to the case where the calcium receptor activator is protamine. It should be noted that an additional description is given below.

Protamine having a calcium receptor-activating function may be used as the active ingredient in the prophylactic or therapeutic agent for diabetes or obesity.
Protamine is particularly suitable for the prophylactic or therapeutic agent for diabetes. The present invention also includes, for example, a prophylactic or therapeutic agent for diabetes comprising protamine as an active ingredient

The prophylactic or therapeutic agent for diabetes or obesity of the present invention may further comprise, in addition to protamine, one kind or two or more kinds of other calcium receptor activators.

When the above-mentioned other calcium receptor activators coexist with protamine, stronger activation on a calcium receptor is observed. The ratio of protamine to the total of other calcium receptor activators in the prophylactic or therapeutic agent for diabetes or obesity of the present invention is not particularly limited as long as a stronger activation on the calcium receptor is possible. For example, the mass ratio of the total of the other calcium receptor activators to the total of protamine is preferably set to 1:100 to 100:1.

The dosage or intake of the prophylactic or therapeutic agent for diabetes or obesity of the present invention may be any amount as long as the amount is effective for therapy or prophylaxis, and is appropriately adjusted depending on the age, gender, body weight, symptom, and the like of a patient. For example, in the case of an oral administration, the total amount of protamine is preferably 0.01 g to 10 g per kg body weight per dose and more preferably 0.1 g to 1 g per kg body weight per dose. The administration frequency is not particularly limited, and the administration may be performed once to several times per day.

The content of protamine in the prophylactic or therapeutic agent for diabetes or obesity of the present invention is not particularly limited as long as the content is suited to the above-mentioned dosage. The content is preferably 0.000001% by mass to 99.9999% by mass, more preferably 0.00001 % by mass to 99.999% by mass, and particularly preferably 0.000 1 % by mass to 99.99% by mass with respect to the dry weight.

The prophylactic or therapeutic agent for diabetes or obesity of the present invention may also be used as a food having a therapeutic or prophylactic effect on diabetes or obesity, for example, a food in a container or a package indicating that the agent has a prophylactic or therapeutic effect on diabetes or obesity. The food comprises protamine in an amount of preferably 0.000001% or more, more preferably 0.00001% or more, or particularly preferably 0.00001% or more and 98% or less. The form of the food is not particularly limited, and the food may be produced in the same production method as a general food and with the same materials as those for the general food except that protamine is blended. Examples of the food include a seasoning, a beverage, a health food, a processed agricultural product, a processed fishery product, and a processed animal product.

### Examples

Hereinafter, the present invention is more specifically described by way of examples. However, the scope of the present invention is not limited to those examples.

### [Reference Example 1] Synthesis of γ-Glu-Val-Gly

Boc-Val-OH (8.69 g, 40.0 mmol) and Gly-OBzl·HCl (8.07 g, 40.0 mmol) were dissolved in methylene chloride (100 ml) and the solution was kept at 0°C. Triethylamine (6.13 ml, 44.0 mmol), HOBt (11-hydroxybenzotriazole, 6.74 g, 44.0 mmol), and WSC·HCl (1-ethyl-3-(3-dimethylaminopropyl)carbodiimide hydrochloride, 8.44 g, 44.0 mmol) were added to the solution, and the mixture was stirred overnight at room temperature. The reaction solution was concentrated under reduced pressure, and the residue was dissolved in ethyl acetate (200 ml). The solution was washed with water (50 ml), a 5% citric acid aqueous solution (50 ml x twice), saturated brine (50 ml), a 5% sodium bicarbonate aqueous solution (50 ml x twice), and saturated brine (50 ml). The organic layer was dried over anhydrous magnesium sulfate, magnesium sulfate was removed by filtration, and the filtrate was concentrated under reduced pressure. The residue was recrystallized from ethyl acetate/n-hexane to obtain Boc-Val-Gly-OBzl (13.2 g, 36.2 mmol) as a white crystal.

Boc-Val-Gly-OBzl (5.47 g, 15.0 mmol) was added to a 4 N HCl/dioxane solution (40 ml), and the mixture was stirred at room temperature for 50 minutes. Dioxane was removed by concentration under reduced pressure, n-hexane (30 ml) was added to the residue, and the mixture was concentrated under reduced pressure. The procedure was repeated three times to quantitatively obtain H-Val-Gly-OBzl·HCl.

### H-val-Gly-OBzl·HCl and Z-Glu-OBzl (5.57 g,15.0 mmol) described above were dissolved in methylene chloride (50 ml), and the solution was kept at 0°C. Triethylamine (2.30 ml, 16.5 mmol), HOBt (1-hydroxybenzotriazole, 2.53 g, 16.5 mmol), and WSC·HCl

(1-ethyl-3-(3-dimethylaminopropyl)carbodiimide hydrochloride, 3.16 g, 16.5 mmol) were added to the solution, and the mixture was stirred at room temperature overnight for 2 days. The reaction solution was concentrated under reduced pressure, and the residue was dissolved in heated ethyl acetate (1500 ml). The solution was washed with water (200 ml), a 5% citric acid aqueous solution (200 ml x twice), saturated brine (150 ml), a 5% sodium bicarbonate aqueous solution (200 ml x twice), and saturated brine (150 ml). The organic layer was dried over anhydrous magnesium sulfate, magnesium sulfate was removed by filtration, and the filtrate was concentrated under reduced pressure. The precipitated crystal was collected by filtration and dried under reduced pressure to obtain
Z-Glu(Val-Gly-OBzl)-OBzl (6.51 g, 10.5 mmol) as a white crystal.

Z-Glu(Val-Gly-OBzl)-OBzl described above (6.20 g, 10.03 mmol) was suspended in ethanol (200 ml), 10% palladium/carbon (1.50 g) was added to the suspension, and a reduction reaction was performed under a hydrogen atmosphere at 55°C for 5 hours. During the reaction, 100 ml in a total volume of water were gradually added. The catalyst was removed by filtration using a Kiriyama funnel, and the filtrate was concentrated under reduced pressure to a half volume. The reaction solution was further filtered through a membrane filter, and the filtrate was concentrated under reduced pressure. The residue was dissolved in a small volume of water, and to the resultant, ethanol was added to precipitate a crystal, and the crystal was collected by filtration and dried under reduced pressure to obtain γ-Glu-Val-Gly as a white powder (2.85 g, 9.40 mmol).

ESI-MS: (M+H)⁺=304.1.
¹H-NMR (400 MHz, D₂O) δ (ppm): 0.87 (3H, d, J=6.8 Hz), 0.88 (3H, d, J=6.8 Hz), 1.99-2.09 (3H, m), 2.38-2.51 (2H, m) 3.72 (1H, t, J=6.35 Hz), 3.86 (1H, d, J=17.8 Hz), 3.80 (1H, d, J=17.8 Hz), 4.07 (1H, d, J=6.8 Hz).

### [Example 1] Function of promoting secretion of CCK

A CCK-producing cell strain STC-1 derived from the mouse small intestine was cultured in a Dulbecco's modified Eagle's medium supplemented with 10% FBS at 37°C in the presence of 5% CC₂. The STC-1 cells were cultured in a 48-well plate for 2 to 3 days until the cells reached a subconfluent state. Prior to the addition of each of samples, the wells were washed with a Hepes B buffer (140 mM NaCl, 4.5 mM KCI, 20 mM Hepes, 1.2 mM CaCl₂, 1.2 mM MgCl₂, 10 mM D-glucose, 0.1% BSA, pH 7.4), 100 µl of a sample solution obtained by dissolving each of the samples in the same buffer were added to the wells, and incubation was performed at 37°C for 60 minutes. γ-Glu-Cys-Gly (Sigma-Aldrich Japan K.K.), γ-Glu-Val-Gly, and cinacalcet were used as the samples. Further, the Hepes B buffer was used as a control. After supernatant collection, the cells were precipitated by centrifugation (800xg, 5 minutes, 4°C), and 80 µl of the supernatant were collected and cryopreserved

After the supernatant had been pretreated with an OASIS cartridge (C 18), the concentration of CCK in the supernatant was measured with an Enzyme immuno assay kit (Phoenix Pharmaceuticals).

FIGS. 1 illustrates the results. The results showed that each of γ-Glu-Cys-Crly, γ-Glu-Val-Gly, and cinacalcet had a function of promoting the secretion of CCK.

### [Example 2] Function of promoting secretion of GLP-1

A GLP-1-producing cell strain GLUTag derived from the mouse large intestine was cultured in a Dulbecco's modified Eagle's medium supplemented with 10% FBS at 37°C in the presence of 5% CO₂. The GLUTag cells were cultured in a 48-well plate for 2 to 3 days until the cells reached a subconfluent state. Prior to the addition of each of samples, the wells were washed with a Hepes B buffer (140 mM NaCl, 4.5 mM KCI, 20 mM Hepes, 1.2 mM CaCl₂,1.2 mM MgCl₂, 10 mM D-glucose, 0.1 % BSA, pH 7.4), 80 µl of a sample solution obtained by dissolving each of the samples in the same buffer were added to the wells, and incubation was performed at 37°C for 60 minutes. γ-Glu-Cys-Gly and cinacalcet were used as the samples. Further, the Hepes B buffer was used as a control. After supernatant collection, the cells were precipitated by centrifugation (800xg, 5 minutes, 4°C), and 70 µl of the supernatant were collected and cryopreserved. The concentration of GLP-1 in the supernatant was measured with a commercially available Enzyme immuno assay kit (Yanaihara Institute Inc.).

FIG. 2 illustrates the results. The results showed that each of γ-Glu-Cys-Gly and cinacalcet had a function of promoting the secretion of GLP-1.

### [Example 3] Function of promoting secretion of GLP-1 (2)

In the same manner as in Example 2, a function of promoting the secretion of GLP-1 was examined using a GLP-1-producing cell strain GLUTag. γ-Glu-Cys and protamine were used as samples.
FIG. 3 illustrates the results. The results showed that each of γ-Glu-Cys and protamine had a function of promoting the secretion of GLP-1.

### [Example 4] GLP-1 secretion test in rats

After male SD rats (8-week-old) had been preliminarily fed, fasted overnight, and subjected to celiotomy under ketamine/xylazine anesthesia. GLP-1-producing cells are mainly distributed in an ileal site. Thus, after a ligated ileal loop (30 cm) had been prepared, a catheter was placed into the ileal mesenteric vein, and blood was collected from the catheter at the time of 0 minute (0 time). A sample (2 mL of deionized water, 20 mg of γ-Glu-Cys/2 mL of deionized water) was administered into the ligated ileum loop, blood was collected at 30, 60, 90, and 120 minutes after administration, respectively, and the plasma GLP-1 concentration was measured with an Enzyme immuno assay kit (manufactured by Yanaihara Institute Inc.).
FIG. 4 illustrates the results. Rats to which γ-Glu-Cys was administered showed a significantly higher elevation of plasma GLP-1 concentration as compared to a water administration group. Therefore, it was confirmed that γ-Glu-Cys had an activity of promoting the secretion of GLP-1 in rats.

### [Example 5] Glucose tolerance test in rats

After male SD rats (7-week-old) had been preliminarily fed (for 3 to 4 days), fasted overnight, and subjected to an oral glucose tolerance test. Tail vein blood before specimen administration was collected, and a sample [glucose solution alone (2 g/kg weight)/glucose solution comprising γ-Glu-Cys (160 mg/kg weight)] was orally administered with a feeding tube. After sample administration, tail vein blood was collected at 15, 30, 60, 90, and 120 minutes, and the plasma glucose concentration was measured with a commercially available kit (manufactured by Wako Pure Chemical Industries, Ltd.).
FIG. 5 illustrates the results. At 15 and 30 minutes after glucose administration, rats to which γ-Glu-Cys was administered showed a significantly lower blood glucose concentration value as compared to a group to which a glucose solution was administered alone. Therefore, it was found that γ-GIu-Cys had a function of suppressing the elevation of plasma glucose. It is understood that the calcium receptor activator having an activity of promoting the secretion of GLP-1 has a function of suppressing the elevation of blood sugar.

### Industrial Applicability

The present invention provides a prophylactic or therapeutic agent for diabetes or obesity, which is highly safe to the living body.

## Claims

1. A prophylactic or therapeutic agent for diabetes or obesity, comprising a calcium receptor activator.

2. The prophylactic or therapeutic agent for diabetes or obesity according to claim 1, wherein the calcium receptor activator is one or more kinds selected from the group consisting of γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-val, γ-Glu-Om, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), cinacalcet, and an analogous compound of cinacalcet.

3. The prophylactic or therapeutic agent for diabetes or obesity according to claim 2, wherein X represents Cys, Cys(SNO), Cys(S-allyl), Gly, Cys(S-Me), Abu, or Ser, and Y represents Gly, Val, Glu, Lys, Phe, Ser, Pro, Arg, Asp, Met, Thr, His, Om, Asn, Cys, or Gln.

4. The prophylactic or therapeutic agent for diabetes or obesity according to claim 2 or 3, wherein the calcium receptor activator is γ-Glu-Val-Gly, γ-Glu-Cys-Gly, or cinacalcet.

5. The prophylactic or therapeutic agent for diabetes or obesity according to claim 2, wherein the calcium receptor activator is γ-Glu-Cys.

6. The prophylactic or therapeutic agent for diabetes or obesity according to claim 1, wherein the calcium receptor activator is protamine.

7. A food for prophylaxis or treatment of diabetes or obesity, comprising γ-Glu-Cys-Gly in an amount of 0.000001% or more.

8. A food for prophylaxis or treatment of diabetes or obesity, comprising γ-Glu-Cys in an amount of 0.000001 % or more.

9. A food for prophylaxis or treatment of diabetes or obesity, comprising protamine in an amount of 0.00000 1 % or more.
